(19) Europäisches Patentamt
European Patent Office
Office européen des brevets

(11) Publication number: **0 383 613 A2**

(12) **EUROPEAN PATENT APPLICATION**

(21) Application number: **90301678.0**

(51) Int. Cl.[5]: **C07D 307/12**

(22) Date of filing: **16.02.90**

(30) Priority: **16.02.89 JP 35080/89**
**17.02.89 JP 36222/89**
**18.04.89 JP 96428/89**
**17.05.89 JP 121581/89**

(43) Date of publication of application:
**22.08.90 Bulletin 90/34**

(84) Designated Contracting States:
**BE CH DE FR GB LI NL**

(71) Applicant: **Tosoh Corporation**
**4560, Oaza Tonda**
**Shinnanyo-shi Yamaguchi-ken(JP)**

(72) Inventor: **Tokunaga, Takumi**
**2591 Oaza-Tonda**
**Shinnanyo-shi, Yamaguchi-ken 746(JP)**
Inventor: **Kagawa, Takumi**
**6-1-317, Madokoro 4-chome**
**Shinnanyo-shi, Yamaguchi-ken 746(JP)**
Inventor: **Watanabe, Hiroyuki**
**5-5-405, Madokoro 4-chome**
**Shinnanyo-shi, Yamaguchi-ken 746(JP)**
Inventor: **Tsuzuki, Kenji**
**8-38, Sakane-cho**
**Shinnanyo-shi, Yamaguchi-ken 746(JP)**
Inventor: **Ito, Mikio**
**484-2, Oaza-Shimogami**
**Tokuyama-shi, Yamaguchi-ken 746(JP)**

(74) Representative: **Kearney, Kevin David Nicholas et al**
**KILBURN & STRODE 30 John Street**
**London, WC1N 2DD(GB)**

# (54) Optically active lactate, derivatives thereof and processes of producing same.

(57) Novel optically active lactate derivatives and manufacturing processes thereof are disclosed. The present invention provides optically active tetrahydrofurfuryl 2-(2S)-hydroxypropionate; and optically active lactate derivative represented by the formula [I]

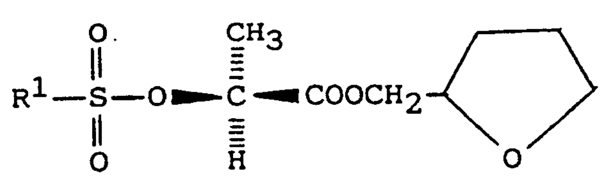

[I]

(wherein $R^1$ represents $C_1$ - $C_3$ alkyl, benzyl, non-substituted phenyl, or mono-, di-, or tri-substituted phenyl substituted with methyl, isopropyl, methoxy, acetylamino, trifluoromethyl, nitro, cyano, fluorine, chlorine, bromine or iodine); optically active tetrahydrofurfuryl 2-chloropropionate; and an optically active hydroxyphenoxypropionate represented by the formula [III].

EP 0 383 613 A2

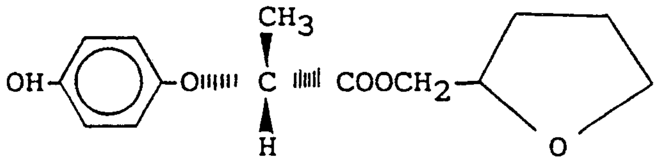

[III]

## Optically Active Lactate, Derivatives Thereof and Processes of Producing the Same

### BACKGROUND OF THE INVENTION

#### I. Field of the Invention

This invention relates to an optically active lactate, derivatives thereof and processes of producing the lactate and its derivatives. The optically active lactate and derivatives thereof of the present invention are useful as intermediates in the manufacturing processes of the compounds with herbicidal activity.

#### II. Description of the Related Art

Some optically active propionate derivaties are known to have excellent selective herbicidal activity. Although some manufacturing processes of these compounds are known, they may be easily prepared if a particular optically active lactate or derivatives thereof exists. However, such a particular optically active lactate or derivatives thereof have not yet been obtained.

### SUMMARY OF THE INVENTION

Accordingly, the object of the present invention is to provide an optically active lactate and derivatives thereof which are useful as intermediates in the production processes of herbicides.

Another object of the present invention is to provide processes for producing the optically active lactate and derivatives thereof of the present invention.

That is, the present invention provides optically active tetrahydrofurfuryl 2-(2S)-hydroxypropionate.

The present invention also provides a process of producing optically active tetrahydrofurfuryl 2-(2S)-hydroxypropionate of the present invention, which process comprising reacting L-lactic acid with tetrafurfuryl alcohol.

The present invention further provides an optically active lactate derivative represented by the formula [I]

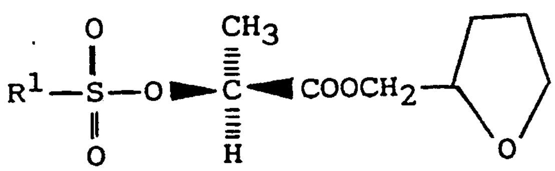

[I]

(wherein $R^1$ represents $C_1$ - $C_3$ alkyl, benzyl, non-substituted phenyl, or mono-, di-, or tri-substituted phenyl substituted with methyl, isopropyl, methoxy, acetylamino, trifluoromethyl, nitro, cyano, fluorine, chlorine, bromine or iodine)

The present invention also provides a process of producing the optically active lactate derivative of the formula [I], which process comprising reacting alkylsulfonic acid halide represented by the formula [II]

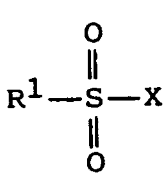

[II]

(wherein $R^1$ represents the same meaning as in formula [I]) and X represents halogen with optically active tetrahydrofurfuryl, 2-(2S)- hydroxypropionate.

The present invention still further provides optically active tetrahydrofurfuryl 2-chloropropionate.

The present invention also provides a process of producing this optically active tetrahydrofurfuryl 2-chloropropionate comprising the steps of reacting optically active tetrahydrofurfuryl lactate with thionyl chloride to prepare chlorosulfinic acid ester of optically active tetrahydrofurfuryl lactate, and then thermally decomposing the resulting chlorosulfinic acid ester of optically active tetrahydrofurfuryl lactate under the presence of a catalyst of an organic base and/or a polar aprotic solvent.

The present invention still further provides an optically active hydroxyphenoxypropionate represented by the formula [III].

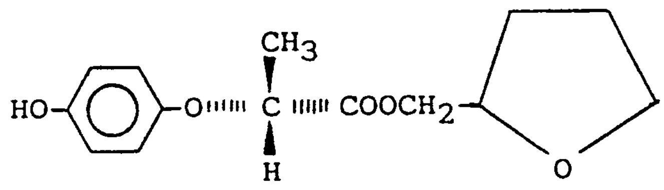

[III]

The present invention also provides a process of producing the optically active hydroxyphenoxypropionate of the formula [III], comprising reacting a hydroquinone derivative of the formula [IV]

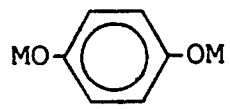

[IV]

(wherein M represents hydrogen, sodium or potassium atom) with a lactate derivative of the formula [V]

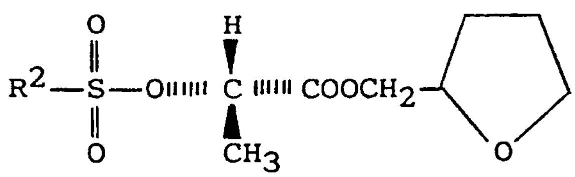

[V]

(wherein $R^2$ represents phenyl, tolyl, mesityl, methyl or trifluoromethyl) under the presence of a base in a non-aqueous solvent.

The present invention still further provides a process of producing the optically active hydroxyphenoxypropionate of the formula [III], comprising reacting optically active 4-hydroxyphenoxypropionic acid with tetrahydrofurfuryl alcohol under the presence of an acid catalyst.

By the present invention, the optically active lactate and derivatives thereof which are useful as intermediates of herbicides with high herbicidal activity and selectivity, as well as production processes thereof are provided.

## DETAILED DESCRIPTION OF THE PREFERRED EMBODIMENTS

As mentioned above, in one aspect of the present invention, optically active tetrahydrofurfuryl 2-(2S)-hydroxypropionate is provided. Although racemic tetrahydrofurfuryl 2-hydroxypropionate is known, optically active tetrahydrofurfuryl 2-(2S)-hydroxypropionate is not known.

This optically active lactate may be prepared in accordance with the following reaction Equation 1:

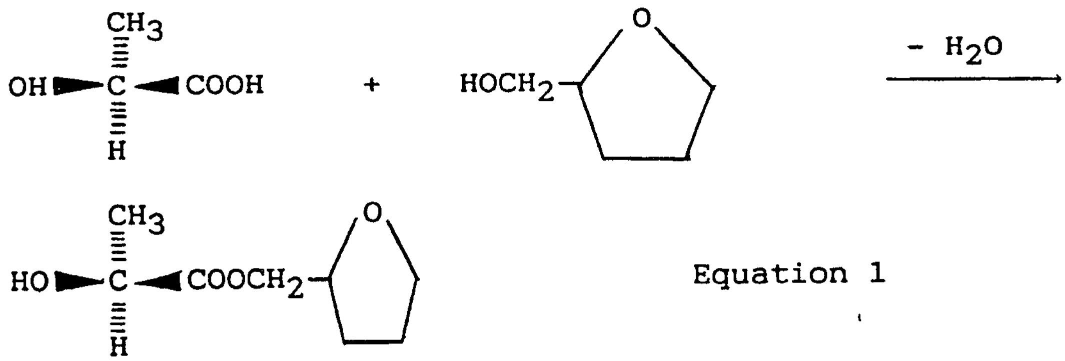


Equation 1

This reaction may be carried out by dehydrating Llactic acid at 70° C - 200° C under reflux in a solvent under the presence of an acid catalyst for 1 - 30 hours, and then reacting the resultant with tetrahydrofurfuryl alcohol at 50° C - 200° for 1 - 30 hours.

In this reaction, preferably, 1 - 10 equivalents of the tetrahydrofurfuryl alcohol and 0.01 - 0.2 equivalents of the acid catalyst may be used with respect to one equivalent of L-lactic acid. The concentrations of L-lactic acid, tetrahydrofurfuryl alcohol and the acid catalyst in the reaction mixture are not critical at all and the reaction may be carried out at any concentration.

Preferred examples of the solvents to be used in the reaction may include aromatic hydrocarbons such as benzene, toluene, xylene and ethylbenzene.

Preferred example of the acid catalyst may include mineral acids such as concentrated sulfuric acid and concentrated hydrochloric acid; organic acids such as aromatic sulfonic acids; Lewis acids such as boron fluoride etherate.

It was confirmed by experiments that optically active tetrahydrofurfuryl 2-(2S)-p-toluenesulfonyloxypropionate can be prepared by reacting the tetrahydrofurfuryl 2-(2S)-hydroxypropionate of the present invention with p-toluenesulfonyl chloride. It was further confirmed by experiments that tetrahydrofurfuryl 2-(2R)-[4-(5-trifluoromethyl-2-pyridyloxy)phenoxy]propionate, tetrahydrofurfuryl 2-(2R)-[4-(3-chloro-5-trifluoromethyl-2-pyridyloxy)-phenoxy]propionate, tetrahydrofurfuryl 2-(2R)-[4-(3,5-dichloro-2-pyridyloxy)-phenoxy]propionate, tetrahydrofurfuryl 2-(2R)-[4-(1,3-dichlorophenoxy)phenoxy]propionate, tetrahydrofurfuryl 2-(2R)-[4-(6-chloro-2-quinoxalinyloxy)phenoxy]propionate can be obtained by reacting the resulting tetrahydrofurfuryl 2-(2S)-p-toluenesulfonyloxypropionate with phenol derivatives, i.e., 2-(4-hydroxyphenoxy)-5-trifluoromethyl pyridine, 2-(4-hydroxyphenoxy)-3-chloro-5-trifluoromethylpyridine, 2-(4-hydroxyphenoxy)-3,5-dichloropyridine, 4-(2,4-dichlorophenoxy)phenol and 2-(4-hydroxyphenoxy)-6-chloroquinoxaline, respectively. It was also confirmed that these compounds had high herbicidal activity against various weeds.

In another aspect of the present invention, an optically active lactate derivative represented by the above-described formula [I] is provided.

In the formula [I], preferred $R^1$ may include methyl, benzyl, non-substituted phenyl and mono-, di- and tri-substituted phenyl substituted with methyl, isopropyl, methoxy, acetylamino, fluorine or chlorine.

Specific preferred examples of the lactate derivatives represented by the formula [I] include:

tetrahydrofurfuryl 2-(2S)-methanesulfonyloxypropionate,
tetrahydrofurfuryl 2-(2S)-ethanesulfonyloxypropionate,
tetrahydrofurfuryl 2-(2S)-propanesulfonyloxypropionate,
tetrahydrofurfuryl 2-(2S)-benzylsulfonyloxypropionate,
tetrahydrofurfuryl 2-(2S)-benzenesulfonyloxypropionate,
tetrahydrofurfuryl 2-(2S)-(4-fluorobenzenesulfonyloxy)propionate,
tetrahydrofurfuryl 2-(2S)-(4-chlorobenzenesulfonyloxy)propionate,
tetrahydrofurfuryl 2-(2S)-(4-bromobenzenesulfonyloxy)propionate,
tetrahydrofurfuryl 2-(2S)-(4-iodobenzenesulfonyloxy)propionate,
tetrahydrofurfuryl 2-(2S)-(2-chlorobenzenesulfonyloxy)propionate,
tetrahydrofurfuryl 2-(2S)-(p- toluenesulfonyloxy)propionate,
tetrahydrofurfuryl 2-(2S)-(o-toluenesulfonyloxy)propionate,
tetrahydrofurfuryl 2-(2S)-(4-nitrobenzenesulfonyloxy)propionate,
tetrahydrofurfuryl 2-(2S)-(N-acetylsulfaniloxy)propionate,
tetrahydrofurfuryl 2-(2S)-(4-methoxybenzenesulfonyloxy)propionate,
tetrahydrofurfuryl 2-(2S)-(2,5-dichlorobenzenesulfonyloxy)propionate,
tetrahydrofurfuryl 2-(2S)-(2,5-dimethoxybenzenesulfonyloxy)propionate,

tetrahydrofurfuryl 2-(2S)-(2,5-dinitrobenzenesulfonyloxy)propionate,
tetrahydrofurfuryl 2-(2S)-(4-chloro-2-nitrobenzenesulfonyloxy)propionate,
tetrahydrofurfuryl 2-(2S)-(4-chloro-3-nitrobenzenesulfonyloxy)propionate,
tetrahydrofurfuryl 2-(2S)-(4-chloro-3-trifluoromethylbenzenesulfonyloxy)propionate,
tetrahydrofurfuryl 2-(2S)-mesitylenesulfonyloxypropionate,
tetrahydrofurfuryl 2-(2S)-(2,4,6-trichlorobenzenesulfonyloxy)propionate,
tetrahydrofurfuryl 2-(2S)-(2,3,4-trichlorobenzenesulfonyloxy)propionate,
tetrahydrofurfuryl 2-(2S)-(2,4,5-trichlorobenzenesulfonyloxy)propionate, and
tetrahydrofurfuryl 2-(2S)-(2,4,6-triisopropylbenzenesulfonyloxy)propionate.

The optically active lactate derivative of the formula [I] may be prepared, for example, in accordance with the following reaction Equation 2:

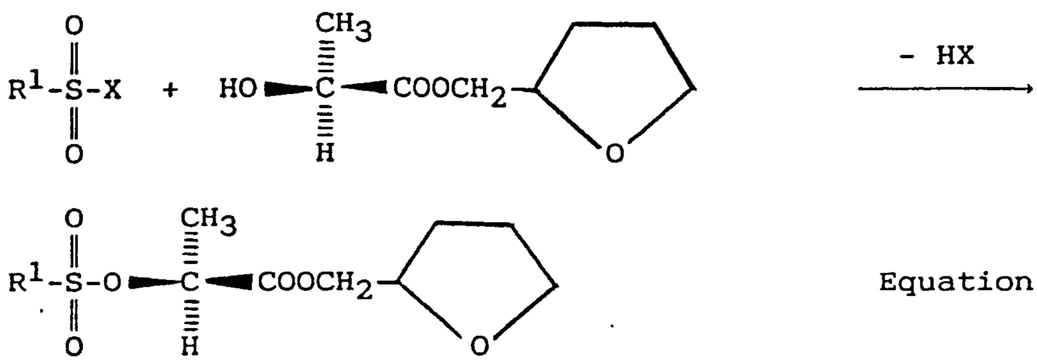


Equation 2

(wherein X represents halogen, preferably fluorine, chlorine, bromine or iodine)

This reaction may be carried out by reacting the reactants at 5° C - 150° C for several minutes to 80 hours in a solvent under the presence of a dehydrogen halide reagent.

In the reaction, preferably, 0.5 - 3 equivalents of optically active tetrahydrofurfuryl 2-(2S)-hydroxypropionate and 1 - 20 equivalents of the dehydrogen halide reagent are used with respect to one equivalent of the alkylsulfonic acid halide of the formula [II].

The concentrations of the alkylsulfonic acid halide, optically active lactate and the dehydrogen halide reagent are not critical at all and the reaction may be carried out at any concentration.

Preferred examples of the solvents to be used in the reaction may include ethers such as ethyl ether, tetrahydrofuran and dioxane; and halogenated hydrocarbons such as carbon tetrachloride, chloroform, dichloromethane and dichloroethane.

Preferred examples of the dehydrogen halide reagents may include tertiary amines such as triethylamine, pyridine, 1,8-diazabicyclo[5,4,0]-undec-7-ene and dimethylaniline; alkali metal hydroxides such as sodium hydroxide and potassium hydroxide; alkaline earth metal hydroxides such as calcium hydroxide; and alkali metal carbonates and alkali metal hydrogen carbonates such as sodium carbonate, potassium carbonate, sodium hydrogen carbonate and potassium hydrogen carbonate.

If the above-mentioned tertiary amine is in the form of a liquid at the reaction temperature, it can be used as the solvent.

The above-mentioned alkali metal hydroxides, alkaline earth metal hydroxides, alkali metal hydrogen carbonates and the alkali metal carbonates may be used in the form of an aqueous solution or solid.

The reaction product may be purified by the conventional method by, for example, extraction with an organic solvent, washing with water and condensation, as well as, if desired, the conventional chromatography.

It was confirmed by experiments that tetrahydrofurfuryl 2-(2R)-[4-(5-trifluoromethyl-2-pyridyloxy)-phenoxy]propionate, tetrahydrofurfuryl 2-(2R)-[4-(3-chloro-5-trifluoromethyl-2-pyridyloxy)phenoxy]propionate, tetrahydrofurfuryl 2-(2R)-[4-(3,5-dichloro-2-pyridyloxy)phenoxy]propionate, tetrahydrofurfuryl 2-(2R)-[4-(1,3-dichlorophenoxy)phenoxy]propionate and tetrahydrofurfuryl 2-(2R)-[4-(6-chloro-2-quinoxalinyloxy)-phenoxypropionate can be obtained by reacting tetrahydrofurfuryl 2-(2S)-p-toluenesulfonyloxypropionate according to the present invention with phenol derivatives, i.e., 2-(4-hydroxyphenoxy)-5-trifluoromethylpyridine, 2-(4-hydroxyphenoxy)-3-chloro-5-trifluoromethylpyridine, 2-(4-hydroxyphenoxy)-3,5-dichloropyridine, 4-(2,4-dichlorophenoxy)phenol and 2-(4-hydroxyphenoxy)-6-chloroquinoxaline, respectively. It was further confirmed that these compounds had high herbicidal activity against various weeds.

In still another aspect of the present invention, optically active tetrahydrofurfuryl 2-chloropropionate is

provided. Although racemic tetrahydrofurfuryl 2-chloropropionate is known (Chemical Abstract, 44, 6568a (1949)), optically active tetrahydrofurfuryl 2-chloropropionate has not yet been obtained.

The optically active tetrahydrofurfuryl 2-chloropropionate may be S-enantiomer or R-enantiomer.

The process of manufacturing optically active tetrahydrofurfuryl 2-chloropropionate will now be described.

First, chlorosulfinic acid ester of optically active tetrahydrofurfuryl lactate is prepared in accordance with the following reaction 3 represented by Equation 3:

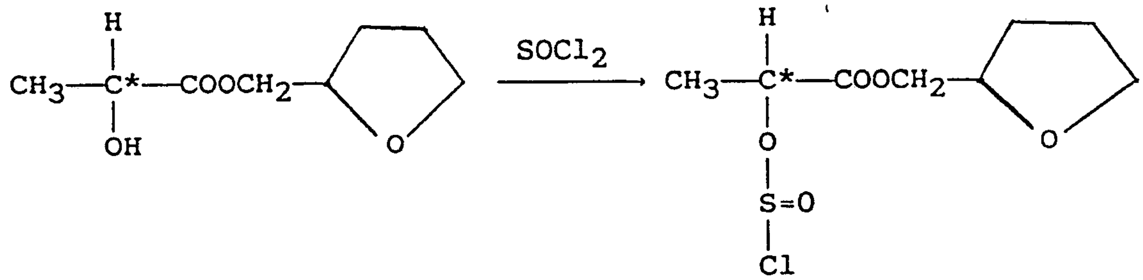


Equation 3

The resulting optically active ester is then thermally decomposed in an aprotic solvent under the presence of a catalytic amount of an organic base to form optically active tetrahydrofurfuryl 2-chloropropionate in accordance with the following reaction 4 represented by

Equation 4:

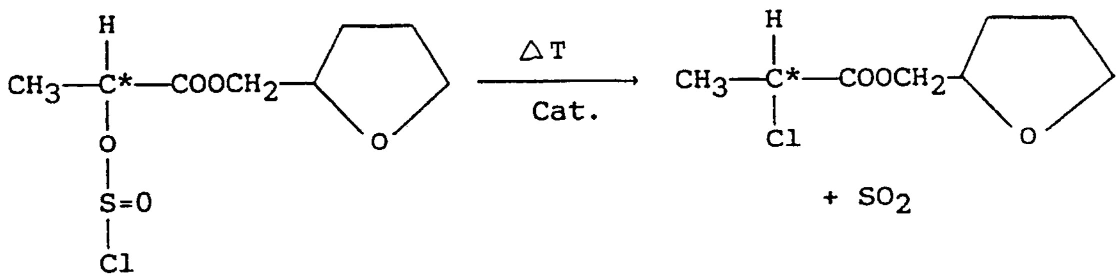


Equation 4

In the Equations 3 and 4, the asteric (*) indicates the asymmetric carbon atom which gives optical activity.

The optically active tetrahydrofurfuryl (2S)-lactate may be prepared as described above and the tetrahydrofurfuryl (2R)-lactate which may also be employed as a reactant may be prepared in the same manner as the tetrahydrofurfuryl (2S)-lactate using D-lactic acid as a starting material.

The reaction 3 may be carried out by supplying a prescribed amount of a solvent and thionyl chloride in a nitrogen-purged reaction vessel and dropping optically active tetrahydrofurfuryl lactate thereon.

Any solvent may be employed in the reaction 3 as long as it does not react with thionyl chloride. Preferred examples of the solvents used in the reaction 3 may include aromatic hydrocarbons such as benzene, toluene and xylene; halogenated hydrocarbons such as dichloromethane, chloroform and carbon tetrachloride; and ethers such as diethyl ether and tetrahydrofuran. Thionyl chloride may be used as the reaction solvent.

In the reaction 3, equimolar thionyl chloride is sufficient with respect to optically active tetrahydrofurfuryl lactate, and use of excess thionyl chloride is not recommended from the viewpoint of economy. Thus, 1.0 - 10.0 times by mole of thionyl chloride with respect to optically active tetrahydrofurfuryl lactate may preferably be employed. In cases where the thionyl chloride is used as a solvent, 5.0 - 10.0 times by mole of thionyl chloride may preferably be used.

The reaction 3 may preferably be carried out at a temperature of 0°C - 50°C. Employing a temperature of higher than 50°C is not preferred because the purity of the chlorosulfinic acid ester of optically active tetrahydrofurfuryl lactate may be reduced because of a side reaction.

Since the hydrogen chloride is generated immediately after mixing optically active tetrahydrofurfuryl lactate and thionyl chloride so that the reaction 3 is completed, the time taken for dropping thionyl chloride at a time interval at which the excess heat generation is prevented is sufficient as the reaction time. However, to vaporize and eliminate the hydrogen chloride dissolved in the system, the reaction mixture may be aged for 1 - 12 hours.

Since the chlorosulfinic acid ester of tetrahydrofurfuryl lactate obtained by the reaction 3 is an unstable compound which is gradually decomposed by the moisture in atmosphere, it is preferred that the reaction product be immediately subjected to the reaction 4 after condensation under reduced pressure at a temperature not higher than 50° C in the nitrogen atmosphere.

Reaction 4 may be carried out as follows: To a reaction vessel equipped with a distillator with an acidic gas trap and a dropping funnel, a high boiling solvent inactive to the reaction, a catalytic amount of the organic base and/or a polar aprotic solvent are supplied. Then the chlorosulfinic acid ester of tetrahydrofurfuryl lactate produced by the reaction 3 is dropped thereto under a temperature and a reduced pressure at which the produced optically active tetrahydrofurfuryl chloropropionate is distilled out.

Although any high boiling solvent may be employed as the high boiling point in the reaction 4 as long as the solvent is inactive to the reaction, those which are liquid at room temperature such as liquid paraffin are preferred. The high boiling solvent may preferably be used in the amount of 10 - 100 times by weight the chlorosulfinic acid ester of tetrahydrofurfuryl lactate. If the amount is less than 10 times by weight, the reaction may not be carried out stably, and if it is more than 100 times by weight, the yield of the desired optically active tetrahydrofurfuryl 2-chloropropionate is reduced.

Preferred examples of the organic base and/or the polar aprotic solvent may include triethylbenzylammonium chloride, tri-($C_5$ - $C_6$)-alkyl-methylammonium chloride, dimethylformamide and hexamethylphosphorylamide.

The amount of the polar aprotic solvent employed in reaction 4 may preferably be 0.1 - 10% by weight of the high boiling solvent.

The thermal decomposition of the chlorosulfinic acid ester of the tetrahydrofurfuryl lactate occurs at a temperature of not lower than 80° C. However, the lower the temperature, the lower the pressure required, so that high degree of vacuum is required for distillating out the desired tetrahydrofurfuryl 2-chloropropionate if the temperature is low and so the practicality of the process is degraded. Thus, in selecting the reaction temperature, the boiling point of the desired product should be taken into consideration. Since a reduced pressure of 0.1 mmHg or higher may be attained by the usual reduction of pressure, a reaction temperature of not lower than 100° C is preferred. However, a temperature of not lower than 180° is not preferred because the racemization of the desired product may occur.

The pressure employed for the reaction 4 is a reduced pressure at which the thermal decomposition of the chlorosulfinic acid ester of the tetrahydrofurfuryl lactate occurs continuously and at which the distillation out of the desired product can be carried out continuously. In the above-described temperature range, 100-180° C, 0.1 mmHg - 10 mmHg is preferred.

Since sulfur dioxide which is a decomposition product is generated as a gas and removed from the system along with the desired product by distillation, it is preferred to equip an absorption tower in which solid KOH or NaOH is packed in the reduced pressure system for removing the $SO_2$ byproduct.

Although the rate of dropping the chlorosulfinic acid ester of tetrahydrofurfuryl lactate may appropriately be selected depending on the concentration of the catalyst, reaction temperature and the pressure, it is preferred that the rate of distillation out of the final product determine the rate of the dropping.

The optically active tetrahydrofurfuryl 2-chloropropionate is an important compound as an intermediate of drugs and agricultural chemicals. By reacting the optically active tetrahydrofurfuryl 2-chloropropionate with a nucleophilic reagent, various optically active tetrahydrofurfuryl 2-substituted propionate derivatives in which the 2-position is substituted with a substituent such as alkyl, vinyl, alkoxy, aryloxy, amino, alkylamino or the like can be prepared.

In still another aspect of the present invention, an optically active hydroxyphenoxypropionate represented by the above-described formula [III] is provided.

The optically active hydroxyphenoxypropionate of the formula [III] may be produced by reacting a hydroquinone derivative of the above-described formula [IV] with a lactic acid derivative of the above-described formula [V] in a non-aqueous solvent in the presence of a base.

Preferred examples of the hydroquinone derivatives represented by the formula [IV] include hydroquinone and sodium and potassium salt thereof.

Preferred examples of the lactic acid derivatives represented by the formula [V] may include benzenesulfonic acid ester, p-toluenesulfonic acid ester, methanesulfonic acid ester, trifluoromethanesulfonic acid ester and mesitylsulfonic acid ester of tetrahydrofurfuryl lactate. Among these, especially preferred are

p-toluenesulfonic acid ester and methanesulfonic acid ester of tetrahydrofurfuryl lactate.

The lactic acid derivative may preferably be used in the amount of 0.1 - 1 equivalent with respect to one mole of the hydroquinone derivative.

Examples of the non-aqueous solvent used in the reaction may include aliphatic hydrocarbons such as hexane and pentane; ethers such as ethyl ether, tetrahydrofuran, monoglyme and diglyme; non-aqueous polar solvents such as dimethylsulfoxide, dimethylformamide and acetonitrile as well as mixtures thereof.

The amount of the solvent may preferably be 5 - 1000 parts by volume, more preferably 10 - 100 parts by volume with respect to one part by volume of the hydroquinone derivative.

Preferred examples of the base employed in the reaction may include alkali metal hydroxides such as sodium hydroxide and potassium hydroxide; carbonates and hydrogen carbonates such as sodium carbonate, potassium carbonate, sodium hydrogen carbonate and potassium hydrogen carbonate; and alkali metal hydrides such as lithium hydride, sodium hydride and calcium hydride. Among these, especially preferred are potassium carbonate, sodium hydroxide, potassium hydroxide and sodium hydride, and most preferred is sodium hydride.

The base is used in the amount of not less than equimolar amount and there is no critical upper limit. The base may preferably be used in the amount of 1 - 5 times by mole the lactic acid derivative.

The reaction may be carried out at a temerature of 0 - 100 °C for several minutes to 48 hours. To obtain higher optical purity, the reaction temperature may preferably be 0 °C - 50 °C.

The optically active hydroxyphenoxypropionate of the formula [III] may also be produced by reacting optically active 2-(4-hydroxyphenoxy)propionic acid and tetrahydrofurfurylalcohol under the presence of an acid catalyst.

The acid catalyst may be, for example, hydrogen chloride and hydrogen bromide, preferably hydrogen chloride.

Although the amount of the acid catalyst is not restricted, it is usually used in saturation.

One to ten parts by weight of the tetrahydrofurfuryl alcohol may preferably be used with respect to one part by weight of optically active (2R)-2-(4-hydroxyphenoxy)propionic acid.

This reaction may be completed at a temperature of 0 - 200 °C for several minutes to 48 hours.

The optically active hydroxyphenoxy propionate represented by the formula [III] may be used as an intermediate of phenoxypropionate-based herbicides which are important in agriculture.

The present invention will now be described by way of examples thereof. It should be noted that the examples are presented for the illustration purpose only and should not be interpreted in any restrictive way.

## Example 1

Preparation of tetrahydrofurfuryl 2-(2S)-hydroxypropionate

A mixture of 196 g of L-lactic acid, 200 ml of benzene and 2 ml of concentrated sulfuric acid were placed in a two liter round bottom flask and a water quantification receiving vessel equipped with a condenser was mounted to the flask. Using an oil bath, the mixture was heated to reflux at 120 °C for 17 hours and 875 g of tetrahydrofurfurylalchol was added to the reaction mixture, followed by stirring at 100 °C for 24 hours. After the reaction mixture was neutralized with sodium acetate, the reaction mixture was distilled under reduced pressure to obtain 295 g of tetrahydrofurfuryl 2-(2S)-hydroxypropionate.

b.p.: 96 - 97 °C/2 mmHg

Refractive Index: $n_D^{25}$ = 1.4541

Optical Rotation: $[\alpha]_D^{20}$ = -12.68 (C = 0.99, EtOH).

Elemental Analysis (%): as $C_8H_{14}O_4$

Found: C; 55.47, H; 8.11, N; 0.00

Calcd.: C; 55.16, H; 8.10, N; 0.00

$^1$H-NMR ($CDCl_3$, δ ppm)

1.25-2.35(7H, m), 3.38-4.62(7H,m)

## Example 2

Preparation of tetrahydrofurfuryl 2-(2S)-hydroxypropionate

A mixture of 195 g of L-lactic acid, 200 ml of benzene and 2 ml of concentrated sulfuric acid were placed in a two liter round bottom flask and a water quantification receiving vessel equipped with a condenser was mounted to the flask. Using an oil bath, the mixture was heated to reflux at 200°C for 10 hours and 874 g of tetrahydrofurfurylalchol was added to the reaction mixture, followed by stirring at 200° for 5 hours. After the reaction mixture was neutralized with sodium acetate, the reaction mixture was distilled under reduced pressure to obtain 200 g of tetrahydrofurfuryl 2-(2S)-hydroxypropionate.
b.p.: 96 - 97°C/2 mmHg
Optical Rotation: $[\alpha]_D^{20}$ = -8.87 (C = 1.02, EtOH)

Example 3

Preparation of tetrahydrofurfuryl 2-p-toluenesulfonyloxypropionate

To a solution of 30.0 g of tetrahydrofurfuryl 2-hydroxypropionate in 26 ml of pyridine contained in a 200 ml round bottom flask, a solution of 27.4 g of p-toluenesulfonyl chloride in 80 ml of chloroform was added dropwise at 0°C for 10 minutes. After the reaction mixture was stirred at room temperature for 16 hours, the reaction mixture was converted to acidic and was extracted with ether. The organic layer was washed with 0.5 N aqueous sodium hydroxide solution and saturated aqueous sodium chloride solution and dried over anhydrous magnesium sulfate. The solvent was then evaporated off under reduced pressure and the residue was purified by silica gel column chromatography (Eluent: n-hexane/ethyl acetate = 3/1 v/v) to obtain 40.6 g of tetrahydrofurfuryl 2-p-toluenesulfonyloxypropionate.
Refractive Index: $n_D^{25}$ = 1.5089
Optical Rotation: $[\alpha]_D^{20}$ = -40.47 (C = 1.01, EtOH)
Elemental Analysis (%): as $C_{15}H_{20}O_6S$
Found: C; 54.96, H; 6.16, N; 0.00
Calcd.: C; 54.86, H; 6.13, N; 0.00
$^1$H-NMR ($CDCl_3$, $\delta$ ppm)
1.48(3H,d), 1.50-2.23(4H,m), 2.40(3H,s), 3.58-4.32(5H,m), 4.90(1H,q), 7.22(2H,d)

Example 4

Preparation of tetrahydrofurfuryl 2-(2S)-p-toluenesulfonyloxypropionate

To a mixture of 8.0 g of tetrahydrofurfuryl 2-hydroxypropionate and a solution of 7.5 ml of triethylamine in 30 ml of methylene chloride contained in a 200 ml round bottom flask, 9.5 g of p-toluenesulfonyl chloride in 30 ml methylene chloride was added dropwise at 0°C for 30 minutes. After the mixture was stirred at room temperature for 4 hours, the reaction mixture was converted to acidic and was extracted with ether. The organic layer was washed with saturated aqueous sodium chloride solution, and the resultant was dried over anhydrous magnesium sulfate and solvent was removed under reduced pressure. The residue was purified by column chromatography in the same manner as in Example 3 to obtain 7.24 g of tetrahydrofurfuryl 2-p-toluenesulfonyloxypropionate.
Optical Rotation: $[\alpha]_D^{20}$ = -40.11 (C = 1.21, EtOH)

Example 5

Preparation of tetrahydrofurfuryl 2-(2S)-methylsulfonyloxypropionate

To a mixture of 8.0 g of tetrahydrofurfuryl 2-(2S)-hydroxypropionate and a solution of 7.5 ml of triethylamine in 30 ml of methylene chloride, was added dropwise 5.7 g of methanesulfonyl chloride in 30 ml of methylene chloride at 0°C for 10 minutes. After the reaction mixture was stirred for 3 hours at room

temperature, the reaction mixture was converted to acidic and then extracted with ether. The organic layer was washed with saturated aqueous sodium chloride solution and the resultant was dried over anhydrous magnesium sulfate. The solvent was then evaporated under reduced pressure and the residue was purified by chromatography in the same manner as in Example 3 to obtain 7.5 g of tetrahydrofurfuryl 2-(2S)-methylsulfonyloxy)propionate.
Refractive Index: $n_D^{25}$ = 1.4590
Optical Rotation: $[\alpha]_D^{20}$ = -44.20 (C=0.78, EtOH)
Elemental Analysis (%): as $C_9H_{16}O_6S$
Found: C; 42.93, H; 6.55, N; 0.00
Calcd.: C; 42.84, H; 6.39, N; 0.00
$^1$H-NMR ($CDCl_3$, $\delta$ ppm)
1.40-2.27(7H,m), 3.12(3H,s), 3.61-4.42(5H,m), 5.13(1H,q)

Example 6

Preparation of tetrahydrofurfuryl 2-(2S)-chloropropionate

To a 2000 ml three-necked flask equipped with a water-quantification receiving vessel, condenser and a stirrer, 200 g of L-lactic acid, 300 ml of benzene and 4.0 g of conc. sulfuric acid were fed. Using an oil bath, the mixture was heated to reflux for 12 hours. After completion of the dehydration, 890 g of 2-tetrahydrofurfurylalcohol was added to the reaction mixture and the resulting reaction mixture was allowed to react at 100 °C for 24 hours.

After neutralizing the obtained reaction mixture with sodium acetate, the reaction mixture was distilled under reduced pressure to obtain 293 g of tetrahydrofurfuryl lactate.
b.p.: 96 - 97 °C/2 mmHg
Optical Rotation: $[\alpha]_D^{20}$ = -12.68

On the other hand, 6.11 g of thionyl chloride and 10 ml of dichloromethane were fed in a 50 ml short neck Kjeldahl flask.

To this mixture, 5.0 g of (2S)-(-)-tetrahydrofurfuryl lactate obtained above was added dropwise using a dropping funnel for 1 hour so as not to allow the temperature of the mixture to exceed 40 °C. The reaction happened simultaneously with the dropping and the generation of hydrogen chloride was observed. After the completion of the dropping, the reaction mixture was stirred for another one hour at room temperature.

The solvent and the excess thionyl chloride were removed from the resulting reaction mixture under reduced pressure at room temperature to obtain 9.20 g of light red oil. The oil was used in the subsequent reaction without purification.

In a 100 ml three-necked round bottom flask equipped with a distillator, reduced pressure line and a dropping funnel, 20 ml of liquid paraffin and 0.5 g of triethylbenzylammonium chloride were fed and all of the chlorosulfinic acid ester of optically active tetrahydrofurfuryl lactate obtained in the above reaction was placed in the dropping funnel. The mixture was stirred and was heated to 130 °C in an oil bath. After the pressure was reduced to 0.5 mmHg by operating the vacuum line, the optically active chlorosulfinic acid ester of tetrahydrofurfuryl lactate was dropped from the dropping funnel at a rate of 0.5 ml/min. Upon the dropping, the mixture began to boil vigorously and the product began to be distilled.

After completion of the dropping, the reaction mixture was left to stand for another 30 minutes at 130 to distill out the remaining desired product. After completion of the reaction, 5.09 g of distilate was obtained in the form of colorless liquid.

Analysis with gas chromatography revealed that the purity of the obtained optically active tetrahydrofurfuryl 2-chloropropionate was 98.5% by area and the yield thereof based on the tetrahydrofurfuryl lactate was 90.7%.
Refractive Index: $n_D^{20}$ = 1.4578
Optical Rotation: $[\alpha]_D^{20}$ = +8.92 (C=1.06, acetone)
Elemental Analysis (%): as $C_8H_{13}O_3Cl$
Found: C; 49.78, H; 6.83, N; 0.00
Calcd.: C; 49.87, H; 6.80, N; 0.00
$^1$H-NMR ($CDCl_3$, $\delta$ ppm)
4.95-3.52(6H,m), 2.20-1.45(7H,m)

Example 7

By the same manner as in Example 6, tetrahydrofurfuryl (2R)-(+)-lactate was obtained using D-lactic acid as a starting material in place of L-lactic acid.
Optical Rotation: $[\alpha]_D^{20}$ = +11.87

Using the same apparatus used in Example 6, 4.8 g of the thus obtained tetrahydrofurfuryl (2R)-(+)-lactate and 10.0 g of thionyl chloride were reacted under the absence of a solvent at a temperature of not higher than 50° C. The resultant was treated as in Example 6 to obtain 8.40 g of chlorosulfinic acid ester of tetrahydrofurfuryl (2R)-(+)-lactate as an oil. The obtained oil was used as it is without purification.

In a 100 ml three-necked round bottom flask equipped with a distillator, reduced pressure line and a dropping funnel, 20 ml of liquid paraffin and 0.5 g of hexamethylphosphoramide were placed and all of the chorosulfinic acid ester of the optically active tetrahydrofurfuryl lactate in the above reaction was supplied to the dropping funnel. While stirring the mixture, the mixture was heated to 150° C in an oil bath. After the pressure in the reaction vessel was reduced to 1.0 mmHg by operating the vacuum line, the chlorosulfinic acid ester of the optically active tetrahydrofurfuryl lactate was dropped at a rate of 0.5 ml/min. Thereafter, the resulting mixture was left to stand under the same conditions for another 30 minutes. After the completion of the reaction, 5.05 g of distilate was obtained as a colorless liquid.

Analysis with gas chromatography revealed that the purity of the thus obtained optically active tetrahydrofurfuryl 2-chloropropionate was 97.5% by area and the yield based on the tetrahydrofurfuryl lactate was 92.7%.
Refractive : Index: $n_D^{20}$ = 1.4575
Optical Rotation: $[\alpha]_D^{20}$ = -8.62 (C=0.99, acetone)
Elemental Analysis (%): as $C_8H_{13}O_3Cl$
Found: C; 49.52, H; 6.72, N; 0.00
Calcd.: C; 49.87, H; 6.80, N; 0.00
$^1$H-NMR ($CDCl_3$, δ ppm)
4.95-3.52(6H,m), 2.20-1.45(7H,m)

Example 8

To 13.4 g of hydroquinone in 50 ml of dimethylsulfoxide, 1.22 g of sodium hydride (60% purity) was added in nitrogen atmosphere. To this mixture, a solution of 10 g of (2S)-2-(p-tosyloxy)propionic acid tetrahydrofurfuryl ester in 10 ml of dimethylsulfoxide was added dropwise. The resulting reaction mixture was distilled at 40° C under reduced pressure and 50 ml of benzene was added to the residue, followed by dropwise addition of 50 ml of 1 N hydrochloric acid to the resulting solution. After separating the organic layer, the aqueous layer was extracted 4 times with 50 ml of benzene and the extracts were combined with the organic layer separated before. The resulting organic layer was dried over anhydrous sodium sulfate and was subjected to a silica gel column chromatography (Eluent: benzene/ethyl acetate = 10/1 v/v) to obtain 5.1 g of the desired product.
Refractive Index: $n_D^{25}$ = 1.54140
Optical Rotation: $[\alpha]_D^{25}$ = +25.7° (chloroform)
$^1$H-NMR ($CDCl_3$, δ ppm)
1.58(3H,d,J=7), 1.1-2.1(1H,m), 3.5-4.4(5H,m), 4.7(1H,q,J=7), 5.85(1H,s), 6.70(4H,dd)

Example 9

To 10.0 g of hydroquinone in 30 ml of hexane, was added 1.22 g of sodium hydride under stirring in nitrogen atmosphere, and the resulting mixture was stirred for another 30 minutes at room temperature. To the reaction mixture, 50 ml of dimethylsulfoxide was added and the resulting mixture was stirred for another 30 minutes. Then 10 g of (2S)-7-(p-tosyloxy)propionic acid tetrahydrofurfuryl ester was added to the resulting solution. The same procedure as in Example 8 was repeated and 6.5 g of the desired product was obtained.
Optical Rotation: $[\alpha]_D^{25}$ = +33.4° (chloroform)

Example 10

The same procedure as in Example 8 was repeated except that potassium carbonate was used as the base to obtain 3.7 g of the desired product.
Optical Rotation: $[\alpha]_D^{25}$ = +32.8° (chloroform)

Example 11

To a solution consisting of 9.1 g of (2S)-2-(4-hydroxyphenoxy)propionic acid and 20.4 g of tetrahydrofurfuryl alcohol, hydrogen chloride gas was blown to saturation. The temperature of the reaction mixture was raised to 110 °C and was stirred at this temperature for 3 hours. The reaction mixture was distilled under reduced pressure and the excess tetrahydrofurfuryl alcohol was recovered. Thereafter, the same procedure as in Example 8 was repeated to obtain 8.37 g of the desired product.
Optical Rotation: $[\alpha]_D^{25}$ = +33.8° (chloroform)

## Claims

1. Optically active tetrahydrofurfuryl 2-(2S)-hydroxypropionate.

2. A process of producing optically active tetrahydrofurfuryl 2-(2S)-hydroxypropionate of claim 1, comprising reacting L-lactic acid with tetrafurfuryl alcohol.

3. An optically active lactate derivative represented by the formula [I]

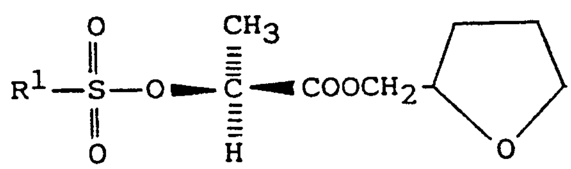

[I]

(wherein $R^1$ represents $C_1$ - $C_3$ alkyl, benzyl, non-substituted phenyl, or mono-, di-, or tri-substituted phenyl substituted with methyl, isopropyl, methoxy, acetylamino, trifluoromethyl, nitro, cyano, fluorine, chlorine, bromine or iodine)

4. A process of producing the optically active lactate derivative of claim 3, comprising reacting alkylsulfonic acid halide represented by the formula [II]

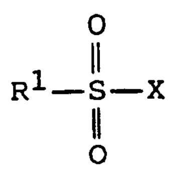

[II]

(wherein $R^1$ represents $C_1$ - $C_3$ alkyl, benzyl, non-substituted phenyl, or mono-, di-, or tri-substituted phenyl substituted with methyl, isopropyl, methoxy, acetylamino, trifluoromethyl, nitro, cyano, fluorine, chlorine, bromine or iodine, and X represents halogen) with optically active tetrahydrofurfuryl 2-(2S)-hydroxypropionate.

5. Optically active tetrahydrofurfuryl 2-chloropropionate.

6. A process of producing optically active tetrahydrofurfuryl 2-chloropropionate of claim 5, comprising the steps of reacting optically active tetrahydrofurfuryl lactate with thionyl chloride to prepare chlorosulfinic acid ester of optically active tetrahydrofurfuryl lactate, and then thermally decomposing the resulting chlorosulfinic acid ester of optically active tetrahydrofurfuryl lactate under the presence of a catalyst of an organic base and/or a polar aprotic solvent.

7. An optically active hydroxyphenoxypropionate represented by the formula [III].

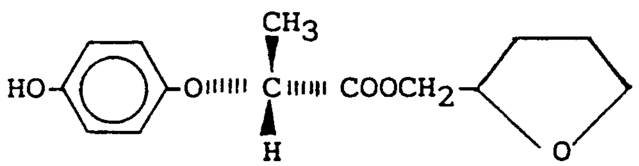

[III]

8. A process of producing the optically active hydroxyphenoxypropionate of claim 7, comprising reacting a hydroquinone derivative of the formula [IV]

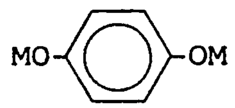

[IV]

(wherein M represents hydrogen, sodium or potassium atom) with a lactate derivative of the formula [V]

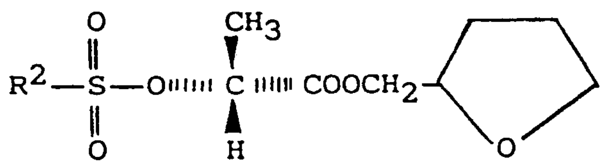

[V]

(wherein $R^2$ represents phenyl, tolyl, mesityl, methyl or trifluoromethyl) under the presence of a base in a non-aqueous solvent.

9. A process of producing the optically active hydroxyphenoxypropionate of claim 7, comprising reacting optically active 4-hydroxyphenoxypropionic acid with tetrahydrofurfuryl alcohol under the presence of an acid catalyst.

10. Tetrahydrofurfuryl 2-(2S)-p-toluenesulfonyloxypropionate,
tetrahydrofurfuryl 2-(2S)-methanesulfonyloxypropionate,
tetrahydrofurfuryl 2-(2S)-ethanesulfonyloxypropionate,
tetrahydrofurfuryl 2-(2S)-propanesulfonyloxypropionate,
tetrahydrofurfuryl 2-(2S)-benzylsulfonyloxypropionate,
tetrahydrofurfuryl 2-(2S)-benzenesulfonyloxypropionate,
tetrahydrofurfuryl 2-(2S)-(4-fluorobenzenesulfonyloxy)propionate,
tetrahydrofurfuryl 2-(2S)-(4-chlorobenzenesulfonyloxy)propionate,
tetrahydrofurfuryl 2-(2S)-(4-bromobenzenesulfonyloxy)propionate,
tetrahydrofurfuryl 2-(2S)-(4-iodobenzenesulfonyloxy)propionate,
tetrahydrofurfuryl 2-(2S)-(2-chlorobenzenesulfonyloxy)propionate,
tetrahydrofurfuryl 2-(2S)-(p-toluenesulfonyloxy)propionate,
tetrahydrofurfuryl 2-(2S)-(o-toluenesulfonyloxy)propionate,
tetrahydrofurfuryl 2-(2S)-(4-nitrobenzenesulfonyloxy)propionate,
tetrahydrofurfuryl 2-(2S)-(N-acetylsulfaniloxy)propionate,
tetrahydrofurfuryl 2-(2S)-(4-methoxybenzenesulfonyloxy)propionate,
tetrahydrofurfuryl 2-(2S)-(2,5-dichlorobenzenesulfonyloxy)propionate,
tetrahydrofurfuryl 2-(2S)-(2,5-dimethoxybenzenesulfonyloxy)propionate,
tetrahydrofurfuryl 2-(2S)-(2,5-dinitrobenzenesulfonyloxy)propionate,
tetrahydrofurfuryl 2-(2S)-(4-chloro-2-nitrobenzenesulfonyloxy)propionate,
tetrahydrofurfuryl 2-(2S)-(4-chloro-3-nitrobenzenesulfonyloxy)propionate,
tetrahydrofurfuryl 2-(2S)-(4-chloro-3-trifluoromethylbenzenesulfonyloxy)propionate,
tetrahydrofurfuryl 2-(2S)-mesitylenesulfonyloxypropionate,
tetrahydrofurfuryl 2-(2S)-(2,4,6-trichlorobenzenesulfonyloxy)propionate,
tetrahydrofurfuryl 2-(2S)-(2,3,4-trichlorobenzenesulfonyloxy)propionate,
tetrahydrofurfuryl 2-(2S)-(2,4,5-trichlorobenzenesulfonyloxy)propionate,
tetrahydrofurfuryl 2-(2S)-(2,4,6-triisopropylbenzenesulfonyloxy)propionate, and
tetrahydrofurfuryl 2-(2R)-[4-(5-trifluoromethyl-2-pyridyloxy)phenoxy]propionate, tetrahydrofurfuryl 2-(2R)-[4-(3-chloro-5-trifluoromethyl-2-pyridyloxy)phenoxy]propionate, tetrahydrofurfuryl 2-(2R)-[4-(3,5-dichloro-2-pyridyloxy)phenoxy]propionate, tetrahydrofurfuryl 2-(2R)-[4-(1,3-dichlorophenoxy)phenoxy]propionate and

tetrahydrofurfuryl 2-(2R)-[4-(6-chloro-2-quinoxalinyloxy)phenoxy]propionate.